# EUROPEAN PATENT APPLICATION

(11) **EP 2 177 241 A1**
(43) Date of publication of application: **21.04.2010**
(21) Application number: 09172735.4
(22) Date of filing: 12.10.2009
(51) Int. Cl.: A61M 16/00, A61B 5/02

(54) **A method and system for synchronizing a patient monitoring device with a ventilator device**

(30) Priority: 20.10.2008 US 254327
(71) Applicant: GENERAL ELECTRIC COMPANY, Schenectady, NY 12345 (US)
(72) Inventor: Tham, Robert, Middleton, WI 53562 (US); Acker, Jaron Matthew, Madison, WI 53703 (US)
(74) Representative: Illingworth-Law, William Illingworth

(57) **Abstract**

A method and system for synchronizing a patient monitoring device with a ventilator device is disclosed herein. The method comprises: accessing a ventilation sequence (110) defined by at least one device setting parameter and initiating assessment of a patient parameter (120) with reference to at least one of the device setting parameter. The method further comprises controlling the progress on the ventilation maneuver (130) based on the patient parameter assessment.

## Description

### FIELD OF THE INVENTION

This invention relates generally to ventilating methods, and more particularly to, a method of synchronizing patient parameter measurement and assessment with a ventilation maneuver.

### BACKGROUND OF THE INVENTION

In a ventilation procedure, it is important to monitor various patient parameters at different stages of a ventilation maneuver to ensure the patient's safety. Before, during and after ventilator maneuvers, it is important to monitor vital signs to determine if the patient will tolerate continuation of the maneuver. For example, during ventilation maneuver monitoring of patient's heart rate or blood pressure (BP) is highly significant as it may indicate high lung pressure that may compromise cardiac blood flow. Conventionally the BP is checked periodically using a non-invasive blood pressure (NIBP) monitor, as and when the clinician commands or repeated periodically such as once every five minutes, irrespective of the ventilation sequence. When the patient parameter is not being monitored continuously, it is essential to check the patient parameter at predefined and early stages of the ventilation maneuver. Currently there is no synchronization between patient parameter measurements and the ventilation maneuvers. Clinician's judgment is used in initiating the patient parameter monitoring or initiating the next level in a ventilation maneuver sequence after the vital signs data is measured. Since the ventilator device and the monitoring device act independently, the lag time or long interval between patient vital sign measurements invariably interferes with the ventilation maneuver. Hence it is advisable to synchronize the measurement or operation of the patient monitoring device with the ventilator device to ensure that patient parameters are being monitored adequately to determine safe progression of the ventilation maneuver.

Further if cross correlation is done manually once the patient parameters or contraindications are detected, that data still needs to be analyzed by a clinician, and based on the same, further progress on ventilation maneuver may be decided. If the contraindications are significant, the ventilation maneuver may need to be aborted. A delay might occur in identifying current status of a patient parameter or interpreting the impacts of contraindications indicated by the patient parameter. Hence it is important to measure and analyze patient parameters quickly with minimum human intervention.

In an example, patients under anesthesia have been reported to experience atelactasis, or lung collapse. Alveolar lung recruitment is a ventilation procedure used to reopen these partially collapse lungs to improve gas exchange, reduce shear forces on repeated opening and closing of lung tissue that may lead to ventilator induced lung injury (VILI). In an alveolar lung recruitment, the ventilator pressurizes the lung to values above typical inspired pressures. While in most patients these higher pressures have no clinical sequalae, it can reduce blood flow in cardiovascular compromised or hypovolemic patients, resulting in low blood pressures and compensatory tachycardia. It is therefore, a good clinical practice to monitor blood pressure or blood flow of the patient during an alveolar recruitment maneuver. As mentioned earlier, NIBP instrument is most commonly used to measure blood pressure but readings are only obtained intermittently as each measurement requires blood circulation to the forearm to be occluded. Typically readings are measured once every 5 minutes, and occasionally once a minute during critical events. The infrequent reading delays the progression of the procedure for alveolar recruitment maneuver. Furthermore, it is inappropriate to prolong the maneuver to wait for a vital signs reading and risk the patient exposure to the elevated ventilation pressures. It would be, therefore, advantageous to develop a device and method that would synchronize these activities and permit the alveolar recruitment to be performed expeditiously along with timely measurement of the vital signs to assess the patient tolerance while the ventilation procedure progresses.

In summary, there exists a need to provide a method and system for synchronizing the operation of a patient monitoring device with a ventilator device.

### SUMMARY OF THE INVENTION

The above-mentioned shortcomings, disadvantages and problems are addressed herein which will be understood by reading and understanding the following specification.

One embodiment of the present invention provides a ventilation method. The method comprises: accessing a ventilation maneuver having a set of ventilation sequence levels that is defined by at least one device setting parameter; initiating assessment of a patient parameter with reference to at least one of the device setting parameter; and controlling the ventilation maneuver based on the assessed patient parameter.

In another embodiment, a method of defining a recruitment maneuver strategy is disclosed. The method comprises: synchronizing an intermittent patient parameter measurement with ventilation sequence of a recruitment maneuver, the synchronization being done with reference to a ventilation sequence level defined by at least one device setting parameter; and measuring at least one patient parameter with reference to a ventilation sequence level.

In yet another embodiment, a lung recruitment method is described. The method comprises: ventilating a patient with a ventilation sequence defined by at least one device setting parameter; synchronizing blood pressure measurement of the patient with at least one ventilator sequence levels; measuring the blood pressure with reference to a desired ventilation sequence level; and controlling ventilation of the patient based on the measured blood pressure.

In yet another embodiment, a ventilating system for ventilating patients is provided. The system comprises: a ventilator device configured for ventilating patients with a ventilating sequence having different ventilation sequence levels defined by at least one device setting parameter; a patient monitoring device associated with the ventilator device, configured for measuring at least one patient parameter at different stages of ventilation; and a controller configured for synchronizing the operation of the patient monitoring device with the ventilator device, by initiating the intermittent measurement of patient parameters based on at least one of the ventilation sequence levels.

In yet another embodiment, the invention provides a computer-readable medium having one or more computer readable media for providing automated ventilation. The medium comprises: a routine for accessing a ventilation sequence that is defined by at least one device setting parameter; and a routine for assessing a patient parameter with reference to at least one of the ventilation sequence levels.

Various other features, objects, and advantages of the invention will be made apparent to those skilled in the art from the accompanying drawings and detailed description thereof.

### BRIEF DESCRIPTION OF THE DRAWINGS

FIG.1 is a flowchart illustrating a ventilation method as described in an embodiment of the invention;
FIG.2 is a flowchart illustrating a ventilation method wherein the patient parameter is measured intermittently, as described in an embodiment of the invention;
FIG.3 is a flowchart illustrating a ventilation method wherein the patient parameter is measured intermittently, as described in another embodiment of the invention;
FIG.4 is a flowchart illustrating a method of defining a recruitment maneuver strategy as described in an embodiment of the invention;
FIG.5 is a flowchart illustrating a ventilation method wherein the patient parameter is being measured continuously, as described in another embodiment of the invention;
FIG.6 is a flowchart illustrating a lung recruitment method as described in an embodiment of the invention;
FIG.7 illustrates a ventilation sequence capable of being used in synchronizing a patient monitoring device with a pressure controlled ventilator device;
FIG.8 is a block diagram of a ventilating system as described in an embodiment of the invention;
FIG.9 is a block diagram of an integrated patient care system as described in an embodiment of the invention; and
FIG.10 is a block diagram of an exemplary embodiment of a ventilation system.

### DETAILED DESCRIPTION OF THE INVENTION

In the following detailed description, reference is made to the accompanying drawings that form a part hereof, and in which is shown by way of illustration specific embodiments that may be practiced. These embodiments are described in sufficient detail to enable those skilled in the art to practice the embodiments, and it is to be understood that other embodiments may be utilized and that logical, mechanical, electrical and other changes may be made without departing from the scope of the embodiments. The following detailed description is, therefore, not to be taken as limiting the scope of the invention.

Embodiments of the present invention improve a ventilation workflow by synchronizing the operation of a patient monitoring device with a ventilator device. To achieve this, an exemplary embodiment of the present invention utilizes a method of automatically initiating assessment of various patient parameters in synchronization with a ventilation sequence, the sequence being defined by a set of device setting parameters. The value of at least one device setting parameter is different from the value of device setting parameter of the ventilation pattern prior to or outside the sequence. For example if the device setting parameter is PEEP, then the value of the same will be different from the value of PEEP used in a ventilation pattern prior to or outside the sequence. In the event of an intermittent patient monitoring schedule, the measurement of the intermittent patient parameter is synchronized with at least one of the device setting parameter and if the patient parameter measurement is done continuously, the assessment of the measured patient parameter is synchronized with at least one of the device setting parameter.

In various embodiments, a ventilating method is disclosed. The real time measurement of patient parameters is included within the ventilation maneuver or the ventilation maneuver is initiated with reference to the timing of the intermittent patient parameter measurement.

In an embodiment of the invention, a method of automatically initiating the measurement of physiological parameters of a patient before, during, and after ventilator maneuvers are disclosed. Different device setting parameters are preempted and with reference to the same an intermittent patient parameter measurement is initiated. The patient parameters indicating contraindication for the maneuver are determined automatically and are analyzed. Based on the measured parameters that indicate the contraindications for the ventilator maneuver, the progress on ventilation maneuver may be decided.

In an embodiment, a method of initiating, synchronizing, correlating, checking and determining various patient parameter measurements within a ventilation maneuver, automatically are disclosed.

In an embodiment, an improved ventilation system is provided. The system automatically identifies various patient parameters that indicate the risk factors of a patient and controls the ventilation maneuver based on the identified patient parameters.

In an exemplary embodiment, an intermittent blood flow or pressure measurement is initiated to assess the patient's tolerance to increased ventilatory pressures during a recruitment maneuver. In an example, synchronizing the patient parameter measurement with the ventilator maneuver is done by initiating the patient parameter assessment in synchronization with a ventilation sequence level defined by device setting parameter.

The terms "ventilation maneuver" and "ventilation sequence" are used in the same meaning, and indicate a sequence having a defined number of breath cycle or duration with at least the value of one device setting parameter being different from the value of device setting parameter of the ventilation pattern prior to or outside the sequence, and based on which the patient is being ventilated over the duration of the ventilation maneuver. The term "device setting parameter" conveys value of device setting parameter in a ventilation maneuver and the value of the same is different from the value of device setting parameter of the ventilation pattern prior to or outside the sequence. The device setting parameters are used to define various levels of the ventilation sequence such as size of breath, time etc. These parameters are device setting parameters of the therapeutic device which is used in ventilating the patient. The examples of the patient parameter, patient monitoring device, ventilating system, device setting parameters etc may not be limited to the few examples mentioned in the specification.

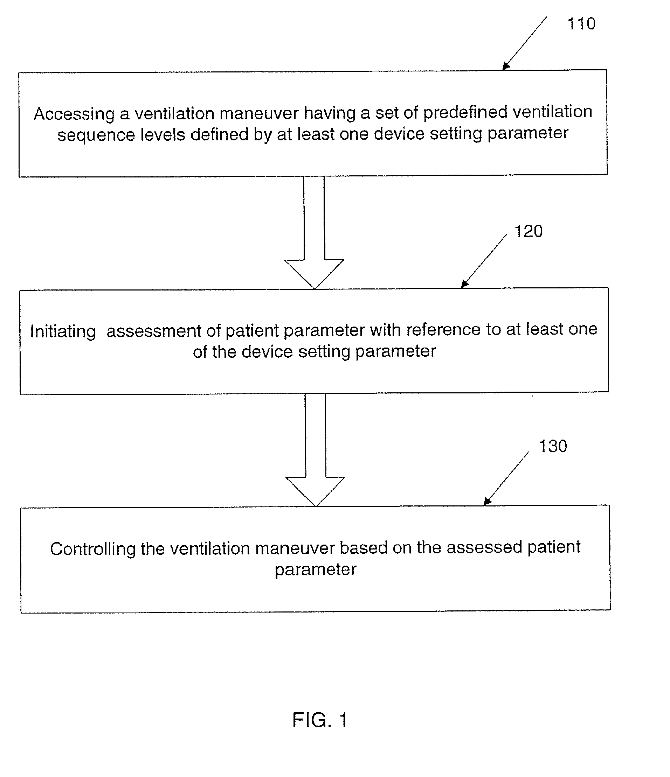

FIG.1 is a flowchart illustrating a ventilation method as described in an embodiment of the invention. At step 110, a ventilation sequence having a set of predefined ventilation sequence levels is accessed. At least one of the levels in the sequence is expected to differ from the ventilation pattern that the patient is being ventilated. Generally, the intent of the difference in the sequence level from the ventilation pattern is to provide the therapeutic treatment or enable other treatment to be performed. The step of accessing the ventilation sequence includes preempting various device setting parameters that define the ventilation sequence levels. Some of the device setting parameters that define the ventilation sequence levels include Pressure end-Expiratory pressure (PEEP), Inspiratory pressure (Pinsp), Tidal volume (TV), inspiratory flow rates, Inspiratory and Expiratory time (Ti, Te), Respiratory rate (RR), and Inspired to Expired ration (I:E ratio).

At step 120, the assessment of the patient parameter measurement is initiated with reference to at least one of the device setting parameter. In an intermittent patient parameter schedule, the intermittent patient parameter measurement is synchronized with the device setting parameters and in case of continuous monitoring the assessment of the measured patient parameter is synchronized with the device setting parameters. Examples of intermittent patient parameters include NIBP and blood flow measurement using dilution method. Examples of continuous monitoring include invasive arterial blood pressure, and invasive Doppler blood flow measurements. The synchronization can be achieved in different ways. In one embodiment, the device setting parameters may be adjusted to adjust the level of the ventilation sequence with reference to the timing of the intermittent patient parameter measurement. This is done based on the requirement of patient parameter measurement. When the patient parameter monitoring is initiated, the device setting parameters are adjusted only after the monitored parameter indicates that the patient can safely tolerate continuation of the maneuver. In another embodiment, the timing of the intermittent patient parameter measurement is adjusted such that it is synchronized with the ventilation sequence levels. In this embodiment, the ventilation sequence is delivered using device setting parameters whose values are predetermined or automatically calculated to achieve the intended purpose of the maneuver, the timing of the intermittent patient parameter measurement may be adjusted. In an embodiment, the patient parameters are measured at different stages of the ventilation maneuver including before, during and after the ventilation maneuver. It is also envisaged that the initiation of the intermittent patient parameter measurement may be based on the synchronization of subsequent repeated measurement with one of the ventilation sequence levels.

At step 130, the ventilation maneuver is controlled based on the assessed patient parameter. If the patient parameter indicates a contraindication of patient in relation to the ventilation maneuver, the ventilation maneuver may be aborted or else the ventilation maneuver may proceed according to the procedure.

In an embodiment, a patient's blood pressure is measured to ensure that ventilation pressure is adequate; the initiation of the cuff inflation to obtain a blood pressure measurement is synchronized with the device setting parameters. In an example, the blood pressure measurement may be synchronized with the PEEP or Pinsp of the ventilation sequence, for example after a first level where the lung pressures are increased but before the much higher recruitment pressure breaths. The early pressure breaths, sometimes referred to as the "precondition breaths", provide the "test" level to ascertain the patient's acceptance of the recruitment pressure breaths. If the pressure measurement is taken during normal patient ventilation, the blood pressure measurement will not offer information whether the patient will tolerate the challenge of a large recruitment pressure breath. If the breath is taken after the recruitment breaths are executed, the patient may have suffered the negative consequence of the elevated pressure breaths. In this case, the synchronization has the effect of taking a reading before the maneuver begins to offer a baseline of the patient during normal ventilation, and compare it with a test breath during the preconditioning recruitment breath, and only on the positive indication of the patient's tolerance of the recruitment breath will be executed. In this case, the synchronization of the NIBP monitor and the ventilation sequence ensures that the timing of the consecutive NIBP measurements are taken in the appropriate sequence levels described taking into consideration the lag time in and minimum time interval between NIBP measurements.

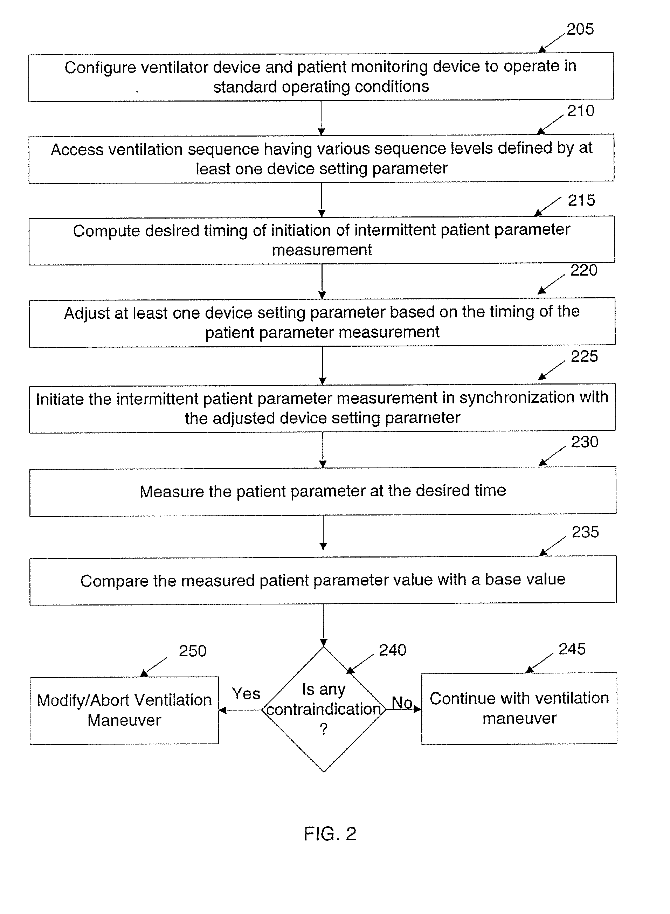

FIG.2 is a flowchart illustrating a ventilation method wherein the patient parameters are monitored intermittently, as described in an embodiment of the invention. At step 205, a ventilator device and a patient monitoring device are configured to operate in standard operating conditions. At step 210, a ventilation sequence having various sequence levels is accessed. In an embodiment, the sequence level in the ventilation sequence may be predefined or user settable. A set of device setting parameters defines the ventilation sequence levels. For accessing the ventilation sequence the ventilation maneuver need not be initiated. At step 215, the timing of the intermittent patient parameter measurement is computed. In an example, a clinician may suggest patient parameter measurement based on various aspects. Alternately, some preset time may be set with reference to ventilation sequence or any other related parameter. At step 220, at least one device setting parameter is adjusted with reference to the timing of the intermittent patient parameter measurement. This is done to synchronize the intermittent patient parameter measurement with the ventilation sequence. At step 225, the intermittent patient parameter measurement is initiated such that the patient parameter is measured in synchronization with the adjusted sequence level, defined by the adjusted device setting parameters. At step 230, the patient parameter is measured at the desired time, i.e. the time at which clinician wanted to take the measurement. At step 235, the measured patient parameter is compared with a base value. At step 240 a check is made to confirm whether the patient parameter indicates any contraindication. If the patient parameter is normal or is within the permissible limit, the ventilation sequence may proceed with the maneuver as at step 245. If the patient parameter indicates contraindication conveying that patient may not tolerate continuation of the ventilation sequence level, the ventilation maneuver may be modified or aborted as at step 250.

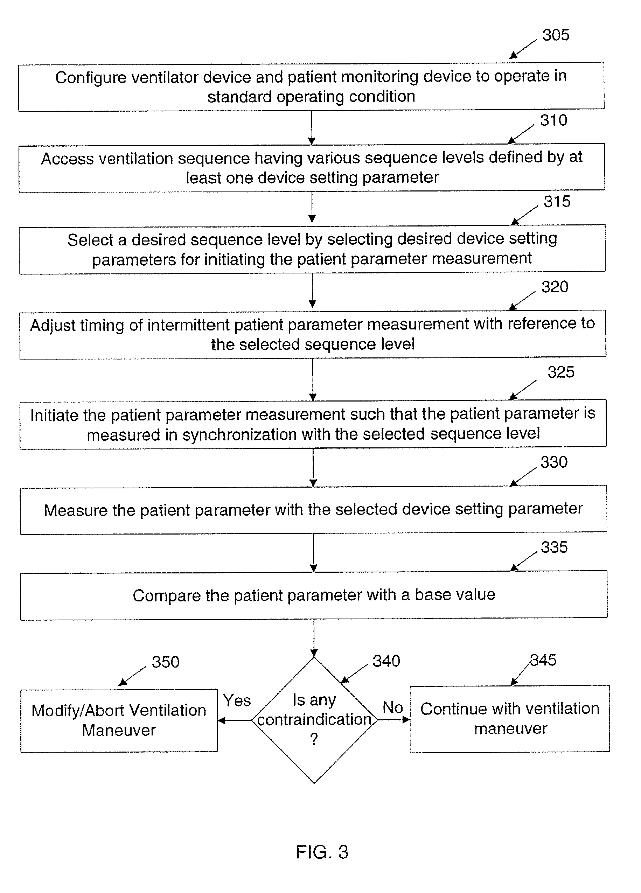

FIG.3 is a flowchart illustrating a ventilation method as described in another embodiment of the invention. At step 305, a ventilator device and a patient monitoring device are configured to operate in standard operating conditions. At step 310, a ventilation sequence having various sequence levels is accessed. In an embodiment the sequence level in the ventilation sequence may be predefined or user settable. A set of device setting parameters defines the ventilation sequence levels. For accessing the ventilation sequence the ventilation maneuver need not be initiated. At step 315, a desired ventilation sequence level is selected by setting desired device setting parameters. In an embodiment, ventilation sequence levels PEEP or Pinsp may be set at a desired value using the device setting parameters. At step 320, the timing of the intermittent patient parameter measurement is adjusted with reference to the selected ventilation sequence level. This is done to synchronize the intermittent patient parameter measurement with the ventilation sequence. At step 325, the intermittent patient parameter measurement is initiated such that the patient parameter is measured in synchronization with selected device setting parameter. At step 330, the patient parameter is measured at the desired sequence level, i.e. the device being set with a desired device setting parameter. At step 335, the measured patient parameter is compared with a base value. In example, the base value could be a normal patient parameter level that conveys that patient is safe or does not show any risk. At step 340 a check is made to confirm whether the patient parameter indicates any contraindication. If the patient parameter is normal or is within the permissible limit, the patient may proceed with the ventilation as at step 345. If the patient parameter indicates contraindication, the ventilation maneuver may be modified or aborted as at 350.

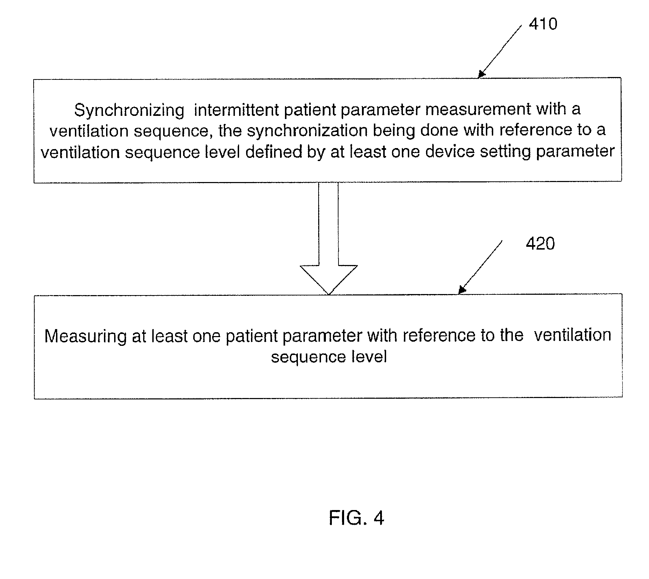

FIG.4 is a flowchart illustrating a method of defining a recruitment maneuver strategy as described in an embodiment of the invention. At step 410, patient parameter measurement is synchronized with a ventilation sequence of the recruitment maneuver. In an embodiment, the synchronization is being done with reference to at least one device setting parameter that defines the ventilation sequence. The ventilation sequence levels may be predefined and the user can have access to information about the device setting parameters corresponding to ventilation sequence levels even before the beginning of the ventilation maneuver i.e. the device setting parameters may be defined by the user in advance. The operation of the intermittent patient parameter measurement device and a ventilating device are synchronized with reference to different ventilation sequence levels set by device setting parameters. The synchronization step may include initiating, correlating and checking of a patient parameter with reference to the ventilator maneuver. In an embodiment, the device setting parameters are adjusted with reference to the timing of the intermittent patient parameter measurement, such that the patient parameter is measured at a certain ventilation sequence level, thereby synchronizing the same. Alternately, the timing of the intermittent patient parameter measurement may be adjusted with reference to the desired device setting parameters, such that the intermittent patient parameter measurement is being done in synchronization with a desired ventilation sequence level set by the desired device setting parameters. At step 420, the patient parameters are measured in synchronization with the ventilation maneuver. The patient parameters are measured with reference to at least one device setting parameters. The measured patient parameters may be compared with a base value and based on the comparison result, the progress on the ventilation maneuver may be decided.

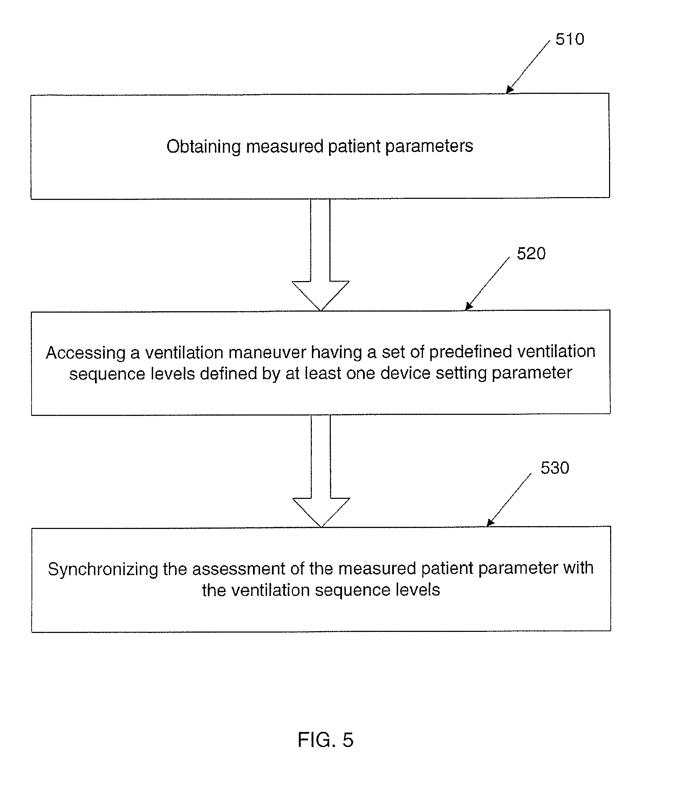

FIG.5 is a flowchart illustrating a ventilation method in an embodiment wherein the patient parameters are measured continuously, as described in an embodiment of the invention. Examples of continuous monitoring include invasive arterial blood pressure, and invasive Doppler blood flow measurements. As mentioned earlier, if the patient parameter measurement is done continuously, then there is no need to synchronize the patient parameter measurement with the ventilation maneuver. At step 510, the measured patient parameters are obtained. The measurement is done continuously and may not be done in synchronization with the ventilation maneuver. At step 520, a ventilation sequence having different levels set by at least one device setting parameter is accessed. At step 530, the assessment of the measured patient parameter is done in synchronization with the ventilation sequence levels.

In an embodiment, patient plethysmogram from a pulse oximeter is used to assess the hemodynamic condition of the patient to accept the ventilation maneuver. While plethysmogram measurements are continuous, the assessment may be synchronized with the ventilation sequence. Further the interpretation of plethysmogram may be done automatically.

In an embodiment, heart rate or variations in heart rate may be measured to identify the changes in lung pressure. The assessment of the patient's tolerance of the ventilation maneuver based on the heart rate measurement may also be synchronized with the ventilation sequence based on device setting parameters.

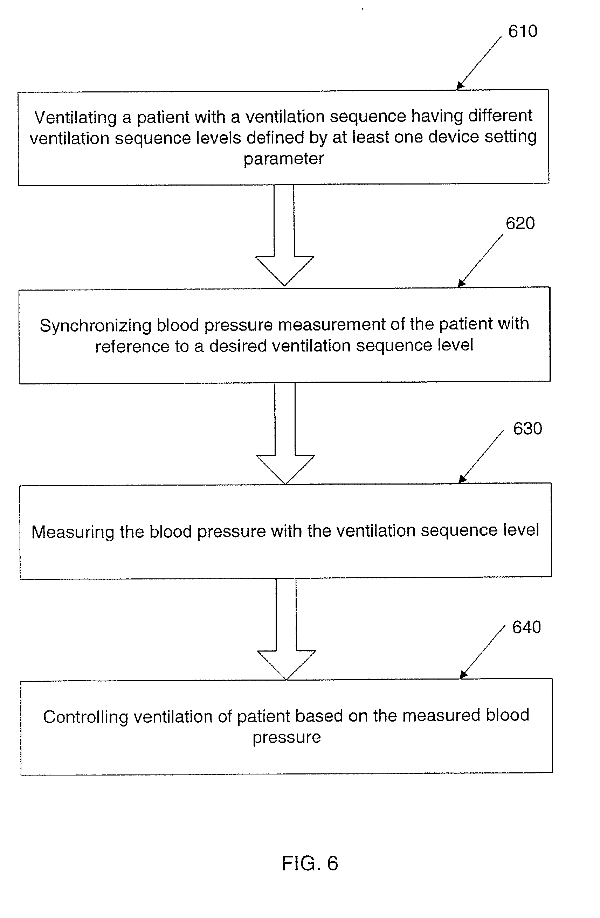

FIG.6 is a flowchart illustrating a lung recruitment method as described in an embodiment of the invention. It is assumed that the blood pressure measurement is taken intermittently. At step 610, a patient is ventilated with a ventilation sequence having different sequence levels, the ventilation sequence being set by different device setting parameters. At step 620, measurement of blood pressure is synchronized with the ventilation sequence that is defined based on at least one level of the device setting parameters. This is done by adjusting the device setting parameters with reference to desired timing of the intermittent blood pressure measurement or by adjusting the timing of the intermittent blood pressure measurement with reference to a desired device setting parameter. At step 630, the blood pressure is measured in synchronization with a ventilation sequence level, typically upon transition between the sequence levels. At step 640, the progression of the ventilation maneuver is controlled based on the measured blood pressure. For example, if the change in blood pressure in response to an early sequence level is unacceptably low, the ventilation maneuver may be aborted.

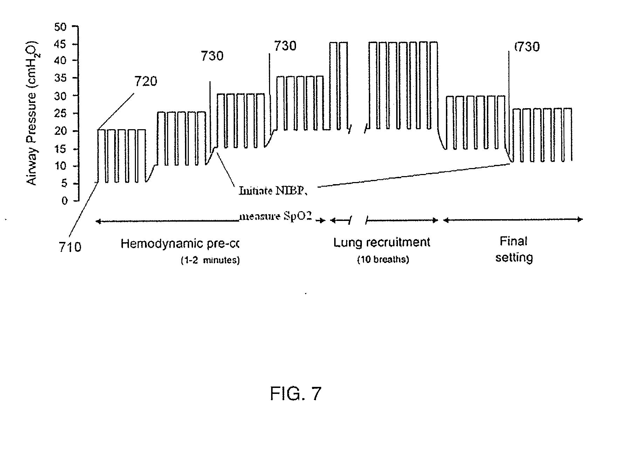

FIG.7 illustrates a ventilation sequence capable of being used in synchronizing a pressure-controlled ventilator device with a patient monitoring device. Ventilation sequence used in a lung recruitment maneuver is illustrated. This maneuver is known as an alveolar recruitment strategy. It is assumed that the patient parameter is not measured continuously. The ventilation sequence is provided as steps in the form of repeated pressure-targeted breath pulses that is different from the breath pattern that the patient was being ventilated. These repeated and stepped breath pulses are predefined or preset at different sequence levels by different device setting parameters. The device setting parameters might include Positive end-expiratory pressure (PEEP), Inspiratory pressure (Pinsp), Tidal volume (TV), Inspiratory flow rates, Inspiratory and Expiratory time (Ti, Te), Respiratory rate (RR), and Inspired to Expired ratio (I:E ratio). Each pressure pulse is configured to have a PEEP pressure 710 as the minimum value and Pinsp 720 as the maximum value. For each step, the values of the pressure pulses will be increased or decreased by a predefined amount depending on the device setting parameters that is varied in accordance to the maneuver. In an embodiment, the intermittent patient parameter measurement can be initiated at different levels, especially when a level change is noticed or anticipated. In an example shown, the intermittent patient parameter measurement is made when the PEEP level changes in ventilation sequence, shown as 730. Similarly, since the pattern of the ventilation sequence is predefined, the level changes in PEEP may be anticipated and the intermittent patient parameter measurement may be synchronized with the same. The patient parameter assessment is synchronized, if the patient parameter is being measured continuously and if the patient parameter is measured intermittently, then the patient parameter measurement is synchronized with the ventilation maneuver to initiate the patient parameter assessment. This invention anticipates such synchronization with other recruitment maneuvers that may last for only one breath or a sequence of breath at only one sequence level. These simpler recruitment maneuvers are commonly called vital capacity maneuver.

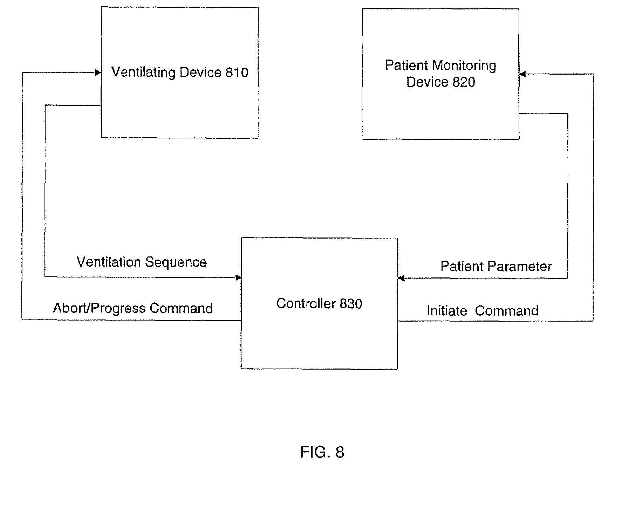

FIG.8 is a block diagram of a ventilating system as described in an embodiment of the invention. The system includes a patient ventilator device 810 and a patient monitoring device 820 configured to measure various patient parameters. The system further comprises a controller 830 configured to synchronize the operation of the patient monitoring device 820 with the ventilator device 810. The ventilator device 810 is configured to ventilate a patient with a ventilating sequence having a set of predefined sequence levels.

The patient monitoring device 820 is configured to monitor patient parameter continuously or at a preset interval. The patient monitoring device 820 is configured to measure various patient parameters that identify contraindications for a ventilation maneuver. The patient parameters include but are not limited to blood pressure (non-invasive and invasive), heart rate, cardiac output, blood gas concentrations, pulse oximetry and electrocardiogram. In an example, the patient monitoring device includes an NIBP (non-invasive blood pressure) monitor, ECG (electrocardiogram) monitor and pulse oximeter.

The controller 830 is configured to synchronize the operation of the patient monitoring device 820 with the ventilator device 810. In an embodiment, it is achieved by synchronizing the timing of patient parameter assessment with at least one of the levels of the ventilation sequence defined by at least one device setting parameter.

In an embodiment, device setting parameters may be known or defined in advance, or may be anticipated. Based on this, the timing of the patient parameter measurement is adjusted such that the patient parameter is measured at a desired time and/or level of the ventilation sequence during the ventilation maneuver. The level or timing of the ventilation sequence parameter may be set by adjusting the device setting parameters. Alternately, based on the intermittent patient parameter measurement lead-time or acceptable repetition rate to obtain a patient parameter measurement, the device setting parameters may be adjusted such that the patient parameter is measured at a particular ventilation sequence level or points in time in the entire ventilation sequence. Upon determining the best synchronized timing of the parameter measurement, the intermittent patient parameter measurement is initiated automatically.

In an embodiment, if the patient parameter is measured continuously, the assessment of the patient parameter by the controller 830 is synchronized with the ventilation sequence level.

The controller 830 may be a part of the ventilator device 810 or the patient monitoring device 820. The controller 830 may be a stand-alone unit configured to receive the ventilation sequence from the ventilator device 810 and the patient parameters from the patient monitoring device 820.

The controller 830 synchronizes the operation of the patient monitoring device 820 by either adjusting the timing of the intermittent patient parameter measurement with reference to at least one device setting parameter or by adjusting the device setting parameters with reference to the timing of the intermittent patient parameter measurement. If the patient parameter is measured continuously the assessment of the measured parameter may be synchronized with the ventilation sequence levels.

The controller 830 may be based on the levels of the ventilation sequence accessed, and may generate an initiate command and send it to the patient monitoring device 820 to initiate the intermittent patient parameter measurement. The controller 830 may receive the ventilation sequence from the ventilator device 810 or the ventilation sequence may be available from a different source. Upon receiving the ventilation sequence, with reference to desired device setting parameters, the controller 830 generates an Initiate command and sends it to the patient monitoring device. Once the patient monitoring device 820 receives the Initiate command, it measures the patient parameters and the measured patient parameter is fed to the controller 830. The controller 830 analyzes the patient parameters for contraindications and based on the analysis, the controller 830 controls the ventilator device. For example, the controller 830 may generate an Abort/Progress command to control the ventilator device 810 based on the measured patient parameter. In an example, the controller 830 may compare the measured patient parameter or the deviation of the measurement from a previous measurement with a predefined or user settable threshold value and based on the same the controller 830 may send a command signal to the ventilator device 810. The command signal may be a command to either proceed with the ventilation or to abort or modify the ventilation maneuver. The controller 830 may include any processor including a microprocessor. In an embodiment, the controller 830 acts as an interface between the patient monitoring device 820 and the ventilator device 810.

In case of a continuous patient parameter measurement, the controller 830 generates a command signal to initiate the assessment of the measured patient parameter with reference to the ventilation sequence level.

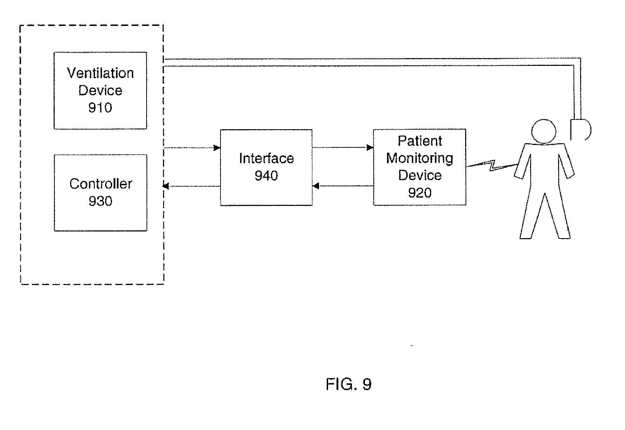

FIG.9 is a block diagram of an integrated patient care system as described in an embodiment of the invention. The integrated patient care system comprises a ventilator device 910 and a patient monitoring device 920. The ventilator device 910 and the patient monitoring device 920 may be integrated using an interface 940 and the interface 940 can be a part of the integrated patient care system. The patient monitoring device is an intermittent patient monitoring device configured to monitor the patient parameters in a desired interval. In an embodiment, the interface 940 facilitates the interaction between the ventilator device 910 and the patient monitoring device 920. A controller 930 may be incorporated in the system to synchronize the operation of the patient monitoring device 920 with the ventilator device 910. The controller 930 is configured to receive the ventilation sequence at different stages of the ventilation maneuver including before, after and during the ventilation maneuver. In an example, a processor associated with the ventilator device 910 is configured to act as a controller 930. The controller 930 synchronizes the operation of the patient monitoring device 920 by either adjusting the timing of the intermittently measured patient parameter with reference to at least one device setting parameters or by adjusting the device setting parameters with reference to the timing of the intermittent patient parameter measurement. In an embodiment, the controller 930 may receive or anticipate different sequence levels and based on the same may control the ventilation maneuver with reference to the intermittently measured patient parameter. In an embodiment, the controller 930 may act as a part of the interface 940 or the interface 940 itself may act as the controller.

In the event of the patient monitoring device 920 being a continuous patient monitoring device, the assessment of the patient parameters measured continuously is done in synchronization with the ventilation maneuver. The controller 930 is configured to synchronize the assessment of the measured patient parameter with the ventilation maneuver.

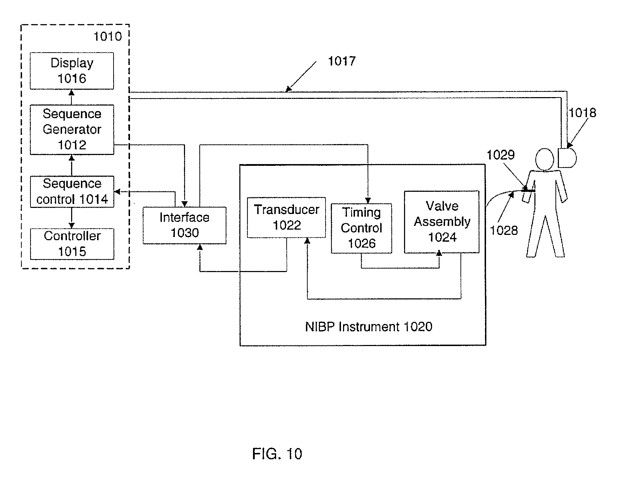

FIG.10 is a block diagram of a ventilation system configured to monitor the patient blood pressure automatically. The ventilation system comprises a ventilator device 1010 having a sequence generator 1012 for generating or accessing a ventilation sequence having a set of predetermined sequence levels, a sequence control 1014 capable of altering different levels of ventilation sequence, a controller 1015 for controlling the operation of the ventilator device 1010 and a display 1016 for displaying the ventilation sequence. The ventilator device 1010 may be further provided with a pipe 1017 and a ventilator interface 1018 to the patient using which a patient could be ventilated. An NIBP monitor 1020 is provided for measuring the patient's blood pressure intermittently or continuously. The NIBP monitor 1020 might include a transducer 1022 to identify the pressure changes in a pressure cuff 1029 and a valve assembly 1024 to control the inflation and deflation of pressure. The pressure cuff 1029 is inflated using the valve assembly 1024 through a tube 1028. The NIBP monitor 1020 further includes a timing control 1026 to adjust the time of initiation of measurement/assessment of the patient parameter. Any processing element present in the NIBP monitor 1020 may be configured to act as the timing control 1026. The system further comprises an interface 1030 for facilitating interaction between the NIBP monitor 1020 and the ventilator device 1010. Ventilator device 1010 is configured to operate initially with a predefined ventilation sequence. The controller 1015 may be provided with the ventilation sequence levels. Once the ventilation sequence levels are accessed, the interface 1030 may generate a control signal to initiate the blood pressure measurement with reference to at least one of the sequence levels decided by at least one device setting parameter. Upon receiving the control signal from the interface 1030, the timing control 1026 may activate the valve assembly 1024 to inflate and deflate the tube 1028 associated with the NIBP monitor 1020. The pressure changes are being sensed by the transducer 1022 and the current blood pressure level is conveyed to the ventilator device 1010 through the interface 1030. Based on the measured blood pressure values, the controller 1015 may control the operation of the ventilator device 1010. The interface 1030 may be a user interface by which a user can interact with the ventilator device 1010 and the NIBP monitor 1020. Through interface 1030 the user may control the operation of the ventilator device 1010 and the patient monitoring device 1020. In an embodiment, the interface 1030 may have a processing unit, which may process the measured Blood pressure or ventilation sequence levels and control the operation of the ventilator device 1010 or the patient monitoring device 1020.

In an embodiment the interface 1030 along with the controller 1015 is used to synchronize the blood pressure monitoring with the ventilation sequence. Based on at least one of the device setting parameter, the operation of the NIBP monitor 1020 is synchronized. Upon synchronizing the same the patient parameter measurement is initiated automatically. In an embodiment, timing of the blood pressure measurement is adjusted based on a desired ventilation sequence level. In another embodiment, the device setting parameters are adjusted using the sequence control 1014 with reference to the timing of the blood pressure measurement, so that the blood pressure measurement is synchronized with at least one of the sequence levels.

In an embodiment, a list of contraindications and the risks involved along with the patient parameters may be accessed. For example, the controller 1015 may access a database having information about different contraindication, permissible limits of patient parameter, risks involved. The controller 1015 may with reference to the same, analyze the measured blood pressure and based on the analysis may automatically abort the ventilation maneuver. Alternately, the system may generate an alarm or provide an indication to the clinician about the contraindications identified.

Some of the advantages of the invention include improving a ventilation workflow and thereby providing quality patient care. The method and system for synchronizing the intermittent patient parameter measurement with the ventilation maneuver improves the efficiency and safety of the ventilation maneuvers. In cases of continuous patient parameter measurement, the assessment of the patient parameter is being synchronized with the ventilation sequence level. Since the parameter measurements are initiated automatically, the contraindications are identified accurately. The automated patient parameter measurement may assist clinician in taking clinical decision or analyzing patient's health condition. Further the measured parameters are analyzed automatically and hence results in improved patient safety. Further the method will reduce the overall time required for the maneuver.

The above-description of the embodiments of the methods and systems has the technical effect of synchronizing the operation of a patient monitoring device with a ventilator device. The synchronization is done based on at least one of the ventilation sequence levels, which is defined by at least one device setting parameter.

Thus various embodiments of the invention describe an improved ventilation workflow and a ventilation system.

As used herein, an element or step recited in the singular and proceeded with the word "a" or "an" should be understood as not excluding plural said elements or steps, unless such exclusion is explicitly recited. Furthermore, references to "one embodiment" of the present invention are not intended to be interpreted as excluding the existence of additional embodiments that also incorporate the recited features.

Exemplary embodiments are described above in detail. The assemblies and methods are not limited to the specific embodiments described herein, but rather, components of each assembly and/or method may be utilized independently and separately from other components described herein. Further the steps involved in the workflow need not follow the sequence in which there are illustrated in figures and all the steps in the work flow need not be performed necessarily to complete the method.

While the invention has been described with reference to preferred embodiments, those skilled in the art will appreciate that certain substitutions, alterations and omissions may be made to the embodiments without departing from the spirit of the invention. Accordingly, the foregoing description is meant to be exemplary only, and should not limit the scope of the invention as set forth in the following claims.

Aspects of the present invention are defined in the following numbered clauses:
1. A ventilation method comprising:
   accessing a ventilation maneuver having a set of ventilation sequence levels that is defined by at least one device setting parameter;
   initiating assessment of a patient parameter with reference to at least one of the device setting parameter; and
   controlling the ventilation maneuver based on the assessed patient parameter.
2. A method as in clause 1, wherein the assessed patient parameter includes: blood pressure or blood flow.
3. A method as in clause 1 or clause 2, wherein the device setting parameter includes: timing of the ventilation sequence.
4. A method as in any one of the preceding clauses, wherein the step of initiating the assessment comprises: initiating an intermittent patient parameter measurement with reference to a device setting parameter.
5. A method as in clause 4, wherein the step of initiating measurement of the patient parameter comprises: adjusting timing of initiation of the intermittent patient parameter measurement with reference to at least one of the ventilation sequence levels.
6. A method as in clause 5, wherein the step of initiating intermittent measurement of a patient parameter comprises: adjusting at least one of the device setting parameter with reference to the timing of intermittent patient parameter measurement.
7. A method as in clause 5, wherein initiating measurement of a patient parameter further comprises: measuring patient parameters at various stages of ventilator maneuver including before, during and after the ventilator maneuver.
8. A method as in any one of the preceding clauses, wherein the step of initiating assessment comprises: assessing continuously measured patient parameter in a patient monitoring process.
9. A method as in any one of the preceding clauses, wherein the step of controlling comprises: determining progress on the ventilation maneuver based on the assessment.
10. A method of defining a recruitment maneuver strategy comprising:
   synchronizing an intermittent patient parameter measurement with ventilation sequence of a recruitment maneuver, the synchronization being done with reference to a ventilation sequence level defined by at least one device setting parameter; and
   measuring at least one patient parameter with reference to a ventilation sequence level.
11. A method as in clause 10, wherein the step of synchronizing comprises: accessing a ventilation sequence defined by a device setting parameter.
12. A method as in clause 10 or clause 11, wherein the step of synchronizing comprises: adjusting the device setting parameters with reference to the timing of the patent parameter measurement.
13. A method as in any one of clauses 10 to 12, wherein the step of synchronizing comprises: adjusting timing of initiation of the intermittent patient parameter measurement with reference to at least one device setting parameter.
14. A method as in clause 13, wherein the step of synchronizing further comprises: initiating, synchronizing, correlating and checking of the intermittent patient parameter measurement with reference to the ventilator maneuver.
15. A method as in clause 13, wherein the synchronization is achieved with reference to device setting parameters including Pressure end-Expiratory pressure (PEEP) or Inspiratory pressure (Pinsp).
16. A lung recruitment method comprising:
   ventilating a patient with a ventilation sequence defined by at least one device setting parameter;
   synchronizing blood pressure measurement of the patient with at least one ventilator sequence level;
   measuring the blood pressure with reference to a desired ventilation sequence level; and
   controlling ventilation of the patient based on the measured blood pressure.
17. A method as in clause 16, wherein the step of synchronizing includes: adjusting at least one device setting parameter with reference to the timing of the blood pressure measurement.
18. A method as in clause 16 or clause 17, wherein the step of synchronizing includes: adjusting the timing of the blood pressure measurement with reference to a device setting parameter.
19. A method as in any one of clauses 16 to 18, wherein the step of controlling comprises: aborting or proceeding with the maneuver based on the measured blood pressure.
20. A ventilating system for ventilating patients comprising:
   a ventilator device configured for ventilating patients with a ventilating sequence having different ventilation sequence levels defined by at least one device setting parameter;
   a patient monitoring device associated with the ventilator device, configured for measuring at least one patient parameter at different stages of ventilation; and
   a controller configured for synchronizing the operation of the patient monitoring device with the ventilator device, by initiating an intermittent measurement of patient parameters based on at least one of the ventilation sequence levels.
21. A system as in clause 20, wherein the monitoring device includes: NIBP monitor, ECG monitor or oximeter.
22. A computer-readable medium having one or more computer readable media for providing automatic ventilation comprising:
   a routine for accessing a ventilation sequence that is defined by at least one device setting parameter; and
   a routine for assessing a patient parameter with reference to at least one of the ventilation sequence levels.
23. A computer readable media as in clause 22, wherein the routine for assessing includes: a routine for automatically initiating an intermittent patient parameter measurement with reference to a ventilation sequence level and a routine for automatically initiating assessment of a continuously measured patient parameter with reference to a ventilation sequence level.
24. A computer readable media as in clause 23, wherein the routine for initiating measurement of a patient parameter further comprises: a routine for adjusting timing of initiation of the intermittent patient parameter measurement with reference to a device setting parameter.
25. A computer readable media as in clause 22, wherein the routine for initiating measurement of patient parameter further comprises: a routine for adjusting the device setting parameter with reference to the timing of patient parameter measurement.

## Claims

1. A ventilation method comprising:
accessing a ventilation maneuver (110) having a set of ventilation sequence levels that is defined by at least one device setting parameter;
initiating assessment of a patient parameter (120) with reference to at least one of the device setting parameter; and
controlling the ventilation maneuver (130) based on the assessed patient parameter.

2. A method as claimed in claim 1, wherein the assessed patient parameter includes: blood pressure or blood flow and the device setting parameter includes: timing of the ventilation sequence.

3. A method as claimed in claim 1 or claim 2, wherein the step of initiating the assessment (120) comprises: initiating an intermittent patient parameter measurement with reference to a device setting parameter.

4. A method as claimed in claim 3, wherein the step of initiating measurement of the patient parameter comprises: adjusting timing of initiation of the intermittent patient parameter measurement with reference to at least one of the ventilation sequence levels.

5. A method as claimed in claim 4, wherein the step of initiating intermittent measurement of a patient parameter comprises: adjusting at least one of the device setting parameter with reference to the timing of intermittent patient parameter measurement.

6. A method as claimed in any one of the preceding claims, wherein the step of initiating assessment (130) comprises: assessing continuously measured patient parameter in a patient monitoring process.

7. A method of defining a recruitment maneuver strategy comprising:
synchronizing an intermittent patient parameter measurement (410) with ventilation sequence of a recruitment maneuver, the synchronization being done with reference to a ventilation sequence level defined by at least one device setting parameter; and
measuring at least one patient parameter (420) with reference to a ventilation sequence level.

8. A method as claimed in claim 7, wherein the step of synchronizing comprises: accessing a ventilation sequence defined by a device setting parameter.

9. A method as claimed in claim 8, wherein the step of synchronizing comprises: adjusting the device setting parameters with reference to the timing of the patent parameter measurement.

10. A method as claimed in any one of claims 7 to 9, wherein the step of synchronizing comprises: adjusting timing of initiation of the intermittent patient parameter measurement with reference to at least one device setting parameter.

11. A method as claimed in any one of claims 7 to 10, wherein the step of synchronizing further comprises: initiating, synchronizing, correlating and checking of the intermittent patient parameter measurement with reference to the ventilator maneuver.

12. A method as claimed in any one of claims 7 to 11, wherein the synchronization is achieved with reference to device setting parameters including Pressure end-Expiratory pressure (PEEP) or Inspiratory pressure (Pinsp).

13. A lung recruitment method comprising:
ventilating a patient (610) with a ventilation sequence defined by at least one device setting parameter;
synchronizing blood pressure measurement of the patient (620) with at least one ventilator sequence level;
measuring the blood pressure (630) with reference to a desired ventilation sequence level; and
controlling ventilation of the patient (640) based on the measured blood pressure.

14. A ventilating system for ventilating patients comprising:
a ventilator device (810) configured for ventilating patients with a ventilating sequence having different ventilation sequence levels defined by at least one device setting parameter;
a patient monitoring device (820) associated with the ventilator device, configured for measuring at least one patient parameter at different stages of ventilation; and
a controller (830) configured for synchronizing the operation of the patient monitoring device with the ventilator device, by initiating an intermittent measurement of patient parameters based on at least one of the ventilation sequence levels.

15. A computer-readable medium having one or more computer readable media for providing automatic ventilation comprising:
a routine for accessing a ventilation sequence that is defined by at least one device setting parameter; and
a routine for assessing a patient parameter with reference to at least one of the ventilation sequence levels.
